# EUROPEAN PATENT APPLICATION

(11) **EP 2 672 412 A1**
(43) Date of publication of application: **11.12.2013**
(21) Application number: 12170796.2
(22) Date of filing: 05.06.2012
(51) Int. Cl.: G06F 19/00

(54) **Method and computer program product for task management on late clinical information**

(71) Applicant: Agfa Healthcare, 2640 Mortsel (BE)
(72) Inventor: Felix, Joost, 2640 Mortsel (BE); Debels, Olivier, 2640 Mortsel (BE); Kieckens, Wannes, 2640 Mortsel (BE)

(57) **Abstract**

When additional clinical information is received (101; 201) by a system for task management on clinical information, the system shall automatically verify (102; 203) if a report already exists for the medical procedure where the additional clinical information belongs to. When a report already exists, the system shall automatically create (103; 204) a task for the author of the report to verify the existing report in view of the additional clinical information received.

## Description

### Field of the Invention

The present invention generally relates to workflow management in clinical applications, e.g. management of the different tasks to be performed by an individual or a team of individuals in a medical imaging environment. Workflow management is for instance important in medical applications such as radiology, where a technician makes medical images of a patient (e.g. an x-ray scan, a CT scan, an MRI scan, an ultrasound scan, etc.), a radiologist interprets the medical images, a transcriptionist produces a report reflecting the radiologists' interpretation, and a clinician receives the report and advises the patient. The individuals involved in the entire workflow may be working at a single facility (e.g. a hospital or imaging centre) or may be working at different locations (e.g. a hospital or enterprise with multiple facilities). In order to govern the different tasks to be performed, a workflow management tool will be deployed and made accessible to the involved individuals at different locations. The current invention in particular concerns a method and tool for workflow management in clinical applications where additional information, e.g. belated medical images, laboratory results, diagnostic information, post-operative information, etc. may arrive in the system at a point in time a report has been generated already for the medical procedure where the additional, belated information belongs to.

### Background of the Invention

The article "From Shared Data to Sharing Workflow: Merging PACS and Teleradiology" from the authors Menashe Benjamin, Yinon Aradi and Reuven Schreiber, published in the European Journal of Radiology 73 (2010) 3-9, describes a system combining storage of medical images and workflow management for radiology. The radiologists or other users of the system may be working on-site or remotely. In order to increase the radiologist's efficiency, the known system produces individual worklists per site, and also a global worklist wherein the tasks of the individual worklists per site are combined for the radiologist.

Since medical images are typically sent between different systems, it may happen that the arrival of a medical image in the known workflow management tool is late. Interpretation and/or diagnosis of the medical images that were available before arrival of the additional image, may have taken place already and the result thereof may be captured in a report that is already available in the system. Traditional workflow management systems either do not notify the author(s) of existing report(s) of the addition of belated information as a result of which the additional information, e.g. a medical image, is not considered and the report may be inaccurate. Alternatively, manually entered notifications may be sent, often through alternative communication channels such as e-mail or sms to notify the involved individuals of the late arrived image or other information. Such manual notifications alerting the people involved are error prone, and often, they are unnecessary because they are addressed to individuals that do not immediately have to perform a task as a result of the late arrival of additional information.

It is an objective of the present invention to disclose a computer-implemented method and tool that overcome the above identified shortcomings of existing tools. More particularly, it is an objective to disclose such a method and tool for use in clinical applications that ensures that additional, belated clinical information is considered in existing reports related to the medical procedure without flooding unnecessary, superfluous and disturbing notifications through various communication channels.

### Summary of the Invention

According to the present invention, the above defined objectives are realized by the method for operating a data processing system for task management on clinical information defined by claim 1, the method comprising:
- receiving by the system additional clinical information in relation to a medical procedure;
- verifying in the system if a report already exists for the medical procedure; and
- automatically creating a task for verification of the report in view of the additional clinical information received.

Thus, the invention consists in checking whether a report was already created for a medical procedure when belated information such as an additional scan arrives in the system. If a report already exists, the system shall automatically create a task for the author of that report to verify if the report is still valid or needs to be updated in view of the additional information. This way, unnecessary notifications are avoided, while it is ensured that the existing report will be reviewed by its author for correctness in view of of the newly available information.

Optionally, as defined by claim 2, the method according to the present invention further comprises:
- applying assignment rules to the task for assigning the task to a person or team using the system.

This way, it can be avoided for instance that a task is assigned to someone no longer working with the system.

Also optionally, as defined by claim 3, the method according to the present invention further comprises:
- sending a notification to inform one or more users of the system on the task; and
- updating for the one or more users each task list whose query matches with the task.

Hence, the users may be notified of creation of the task, e.g. through a pop-up window on their screens, and/or their task lists may be updated automatically to include the new task.

According to another optional aspect, defined by claim 4, the method according to the present invention may further comprise:
- adding comments to the task.

This way, it becomes possible to inform the author of the report for instance of the reason why the task was generated.

Still optionally, as defined by claim 6, the method according to the present invention may further comprise:
- adding escalation rules to the task to be applied when the task is not executed within a set time interval.

This way, non-execution of the verification task within a certain time limit could be brought to a supervisor's attention enabling the latter to re-assign the task.

According to yet another optional aspect defined by claim 6, the method according to the current invention comprises:
- triggering a distribution flow for an updated version of the report.

Hence, based on the outcome of the report verification task, a distribution flow for the updated report could be triggered.

As is further specified by claim 7, the additional clinical information may be a belated medical image.

Optionally, as defined by claim 8, the type of the belated image may be verified and the task for verification of the report may be generated only when the belated image is of a predefined type.

If for instance the DICOM standard is used for communicating medical images between different systems, the present invention may filter on the type of image such that only the arrival of real images leads to the creation of a verification task for existing reports. This way, verification tasks are avoided to be triggered by late arrival of derived images in the system. As a result, the number of unnecessary tasks created automatically is reduced, further increasing the efficiency of the system.

In addition to the method defined by claim 1, the present invention relates to a corresponding data processing system as defined by claim 9, comprising means for carrying out the method.

The current invention in addition also relates to a computer program as defined by claim 10 comprising software code adapted to perform the method.

The invention further relates to a computer readable storage medium as defined by claim 11, comprising the computer program.

### Brief Description of the Drawings

Fig. 1 is a flow chart of a first embodiment of the method according to the present invention; and

Fig. 2 is a flow chart of a second embodiment of the method according to the present invention.

### Detailed Description of Embodiment(s)

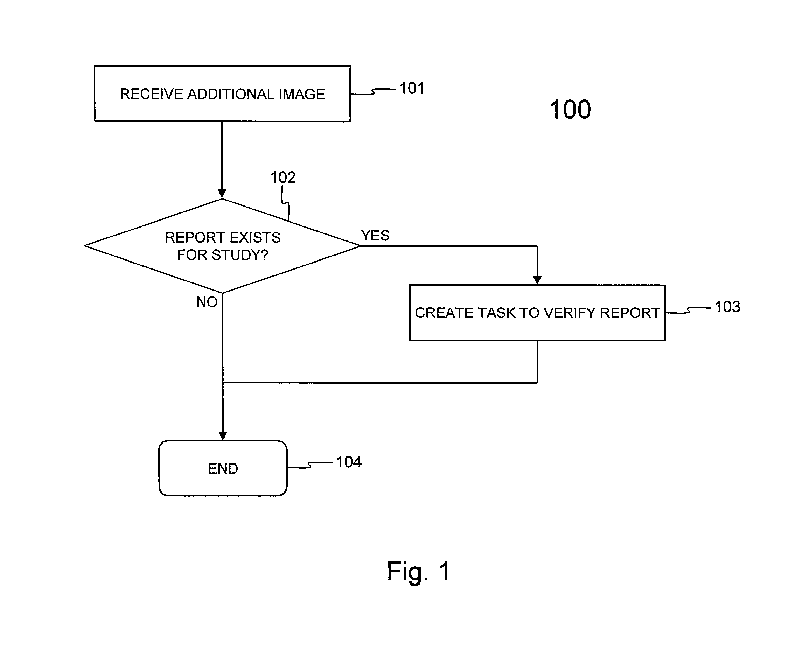

Fig. 1-2 illustrate embodiments of the current invention implemented as part of a software application for workflow management in radiology. Such workflow management application typically has a client-server architecture.

The server part of the application receives queries from the client part, runs these queries on a centralized or distributed database system wherein at least the medical images, medical reports, and tasks are stored and maintained. The server part also notifies the client part in case of changes in the database system that match with one of the queries received from that client, in order to enable the client part to update and refresh the information displayed to the user.

The client part of the application enables the user to configure queries, and it generates the desktops for different types of users and the various screens according to the user's preferences and configuration settings. In the application for radiology, the desktops generated by the client part may for instance comprise a diagnostic desktop for radiologist users, a configuration desktop for administrator users, a transcriptionist desktop for the transcriptionist-user that uses the system to transfer speech recordings into text, a clinician desktop for the clinician-user that reads the medical reports from the radiologist(s) and advises the patient, a technologist desktop for the technician-user that makes the medical images. Each of these users makes use of one or more task lists accessible through his/her desktop for workflow management. Each such task list can be configured, i.e. the data fields to be displayed in a screen as part of the task list can be selected by the user, filtering criteria enabling selection of the relevant tasks for that task list are configurable by the user, and the criteria to be applied for sorting the tasks within the task list can be configured by the user. In the embodiments described in the following paragraphs, a radiologist user is assumed to have authored a report. At a later point in time, additional images are uploaded in the database system by a technician.

Fig. 1 illustrates a basic implementation of the method according to the invention. In step 101, one or several additional medical images are received by the system that is used for workflow management in a medical imaging environment. The additional image(s) may for instance be images resulting from an additional scan, e.g. an x-ray scan, a CT scan, an MRI scan, an ultrasound scan, etc., or they may be processed images, e.g. a 3D reconstruction, an image supplemented with data, etc. In step 102, the system automatically verifies if the additional images belong to a medical procedure for which a report has been produced already. If no report is existing in the system for that medical procedure, the method shall end, as is indicated by 104 in Fig. 1. The received additional images shall be stored together with earlier stored images in order to be processed and/or studied later. In case a report is already existing for that medical procedure, the existing report may be inaccurate or incomplete because it was produced without considering the additional images. In such case, the system shall automatically create a task for the author of the existing report to verify the existing report in view of the additional images. This is indicated by step 103 in Fig. 1. The automatically generated task shall be added to the overall task list in the system and be treated, i.e. notified to the author, escalated, etc. in a manner similar to other tasks. The method again ends at step 104.

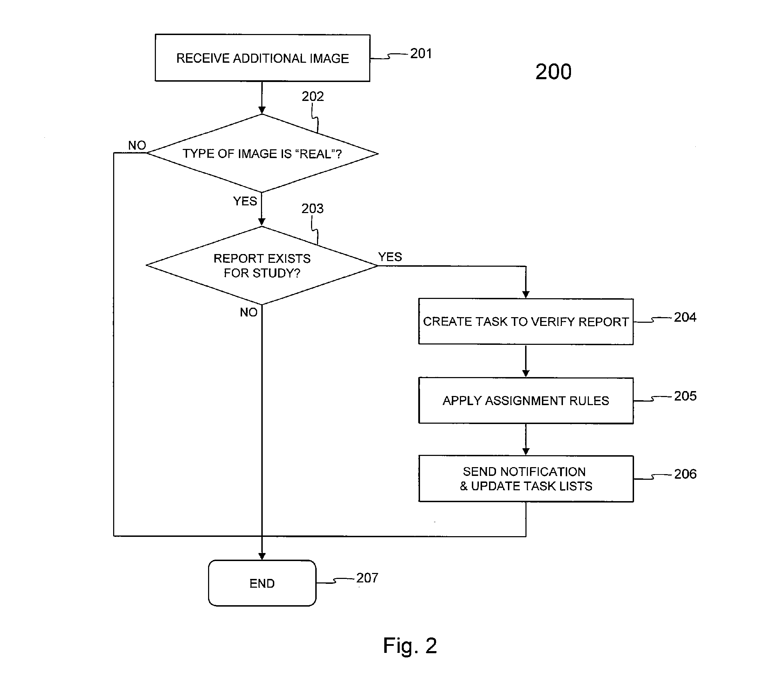

Fig. 2 illustrates a more advanced implementation of the method according to the invention. In step 201, the system again receives one or several additional, belated medical images in relation to a medical procedure for which the system performs task management. In order to reduce the amount of unnecessary tasks generated by the system, the system in step 202 first verifies the type of the additional image(s). If the DICOM (Digital Imaging and Communications in Medicine) standard is used to transfer the additional images to the system, the DICOM metadata may be consulted by the system in order to determine if an additional image is a "real" image, i.e. a first capture, or a processed image, i.e. a second capture that contains for instance data, parameter values, a 3D reconstruction, etc. If the additional image is a second capture, i.e. a derived image obtained through processing of existing images that are already available in the system, the system shall assume that it is not necessary to generate additional tasks and immediately terminate at step 207. If the additional image is a first capture, the system shall automatically verify in step 203 if a report is already existing for the medical procedure where the additional image relates to. In case no report exists in the system for that medical procedure, the method shall again terminate at step 207. The additional image(s) shall then be stored in the system for later evaluation. If however one or several reports exist already for the medical procedure where the additional image belongs to, the system will automatically create a task for the author(s) of each of these reports to verify the existing reports in view of the additional image. This is done in step 204 in Fig. 2. The system thereupon automatically assigns the created task to the person or group of persons that authored the existing report(s). This is done in step 205. In case the author is no longer working or no longer using the system, the verification task shall be assigned to another responsible for the medical procedure according to assignment rules that are predefined in the system. This way, it is avoided that a newly created task will be assigned to a person or team that is no longer using the system. In step 206, the system automatically notifies the user(s) whereto the verification task is assigned. In addition, the system automatically updates all task lists of these user(s) whose query matches with the newly created task. Indeed, in an application for radiology departments for instance, a radiologist shall typically maintain different task lists, like for instance high priority tasks that need to be completed by a close deadline, reading tasks like the interpretation of certain medical images as soon as these images have become available in the system, and sign-off tasks. When the high priority task list is empty, the radiologist shall start to work on the reading tasks. When both the high priority task list and the reading task list are empty, the radiologist will switch to sign-off tasks. It is noticed that tasks may appear in multiple individual lists, or dynamically move from one list to another. A reading task or sign-off task may for instance become a high priority task when its deadline is nearing. Each task list is defined by a query. Tasks that match with the query appear in the task list. Thanks to the current invention, the task for verification of an existing report that is automatically created by the system upon arrival of an additional "real" image, shall appear in the task lists of the assigned persons whose queries match with the created task.

Optionally, more sophisticated embodiments of the method according to the invention could be contemplated. Comments could for instance be attached to the created verification task to explain why the task was created. Such comments could contain a reference or link to the additional image(s) received by the system. The system may also implement escalation rules that define which responsible or supervisor needs to be informed when the task for verification of the existing report is not completed by the assigned person(s) within a given time frame. The system may also automatically trigger a distribution flow as soon as the existing report has been reviewed and eventually has been updated by the person(s) to whom this task was assigned. This way, it is ensured that all persons making use of the report become aware of the existence of a new version of the report that also considers the additional images arrived late in the system.

It is further noticed that although the DICOM standard was mentioned in relation to the embodiments described here above for formatting / reporting medical images, it will be apparent to the person skilled in the art of medical imaging and related task management that the present invention is not limited to the use of this standard. Alternative protocols for formatting and reporting medical images, like for instance HL7 or EDIFACT-EDI standards may be used. The present invention may take benefit of the protocol for filtering if the metadata contain an indication of the type of image, as is the case for DICOM.

It is also remarked that the invention illustrated here above with two embodiments in the field of medical imaging, may be applied more widely to late arrival of any type of clinical information in a task management system. Such clinical information may include laboratory results, diagnostic information, post-operative information, etc. Upon late arrival of such information in the system, i.e. arrival at a point in time where a report already exists for the medical procedure where the newly arrived information belongs to, a task for verification of the existing report(s) can be generated automatically with same advantages as described here above in relation to medical images.

The method according to the invention shall typically be computer-implemented on a system or platform with client-server architecture. The images and tasks are maintained centrally or distributed on one or more servers. Users access their task lists and consult images stored in the system via a client device. A data processing system or computing device that is operated according to the present invention can include a workstation, a server, a laptop, a desktop, a hand-held device, a mobile device, a tablet computer, or other computing devices, as would be understood by those of skill in the art.

The data processing system or computing device can include a bus or network for connectivity between several components, directly or indirectly: a memory or database, one or more processors, input/output ports, a power supply, etc. One of skill in the art will appreciate that the bus or network can include one or more busses, such as an address bus, a data bus, or any combination thereof, or can include one or more network links. One of skill in the art additionally will appreciate that, depending on the intended applications and uses of a particular embodiment, multiple of these components can be implemented by a single device. Similarly, in some instances, a single component can be implemented by multiple devices.

The data processing system or computing device can include or interact with a variety of computer-readable media. For example, computer-readable media can include Random Access Memory (RAM), Read Only Memory (ROM), Electronically Erasable Programmable Read Only Memory (EEPROM), flash memory or other memory technologies, CDROM, digital versatile disks (DVD) or other optical or holographic media, magnetic cassettes, magnetic tape, magnetic disk storage or other magnetic storage devices that can be used to encode information and can be accessed by the data processing system or computing device.

The memory can include computer-storage media in the form of volatile and/or nonvolatile memory. The memory may be removable, non-removable, or any combination thereof. Exemplary hardware devices are devices such as hard drives, solid-state memory, optical-disc drives, or the like. The data processing system or computing device can include one or more processors that read data from components such as the memory, the various I/O components, etc.

The I/O ports can allow the data processing system or computing device to be logically coupled to other devices, such as I/O components. Some of the I/O components can be built into the computing device. Examples of such I/O components include a microphone, joystick, recording device, game pad, satellite dish, scanner, printer, wireless device, networking device, or the like.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A method (100; 200) for operating a data processing system for task management on clinical information, said method comprising:
- receiving (101; 201) by said system additional clinical information in relation to a medical procedure;
- verifying (102; 203) in said system if a report already exists for said medical procedure; and
- automatically creating (103; 204) a task for verification of said report in view of said additional clinical information received.

2. A method (200) according to claim 1, further comprising:
- applying (205) assignment rules to said task for assigning said task to a person or team using said system.

3. A method (200) according to any of the preceding claims, further comprising:
- sending (206) a notification to inform one or more users of said system on said task; and
- updating (206) for said one or more users each task list whose query matches with said task.

4. A method according to any of the preceding claims, further comprising:
- adding comments to said task.

5. A method according to any of the preceding claims, further comprising:
- adding escalation rules to said task to be applied when said task is not executed within a set time interval.

6. A method according to any of the preceding claims, further comprising:
- triggering a distribution flow for an updated version of said report.

7. A method according to any of the preceding claims, wherein said additional clinical information is a belated medical image.

8. A method (200) according to claim 7, wherein a type of said belated image is verified (202) and said task for verification of said report is only generated when said belated image is of a predefined type.

9. A data processing system comprising means for carrying out the method of any of claims 1 to 8.

10. A computer program comprising software code adapted to perform the method of any of claims 1 to 8.

11. A computer readable storage medium comprising the computer program of any of claims 1 to 8.
